# EUROPEAN PATENT SPECIFICATION

(11) **EP 3 320 941 B1**
(45) Date of publication and mention of the grant of the patent: **05.08.2020**
(21) Application number: 17199831.3
(22) Date of filing: 03.11.2017
(51) Int. Cl.: A61M 16/06, A61M 16/08

(54) **ARTIFICIAL RESPIRATION SYSTEM**
SYSTEM ZUR KÜNSTLICHEN BEATMUNG
SYSTÈME DE RESPIRATION ARTIFICIELLE

(30) Priority: 14.11.2016 IT 201600114357
(43) Date of publication of application: 16.05.2018
(73) Proprietor: Intersurgical S.P.A., 41037 Mirandola, Modena (IT)
(72) Inventor: NAVALESI, Paolo, 20149 MILANO (IT); LONGHINI, Federico, 28021 BORGOMANERO (NO) (IT); ROSSI, Paolo, 41037 MIRANDOLA (MO) (IT)
(74) Representative: Corradini, Corrado

(56) References cited:
- EP-A1- 2 684 578
- EP-A2- 1 279 411
- US-A1- 2007 113 848
- US-A1- 2007 113 848
- US-A1- 2012 125 338

## Description

### TECHNICAL FIELD

The present invention relates to a system for the artificial respiration of patients.

In particular, the invention relates to a system that comprises a helmet for mechanical ventilation in continuous positive airway pressure (CPAP) and a nasal cannula for oxygen therapy.

### PRIOR ART

As is known, for the artificial respiration, such as non-invasive ventilation (NIV) or mechanical ventilation in continuous positive airway pressure (CPAP), of patients in general, devices are used which include helmets that, in general, consist of a substantially cylindrical container body, are provided with a collar consisting of a thin membrane of an elastically yieldable plastic material which is coupled to the patient's neck to provide the seal, and input and output openings, respectively, for the inlet of air and oxygen to breathe and for the outlet of breathing exhaust gases (CO₂).

These helmets, as disclosed in documents EP2684578 and EP1797925, are used in oxygen therapy and in the ventilation of patients with continuous positive pressure, the so-called CPAP and NIV, in order to allow breathing in patients who would not be able to breathe independently.

US 2007/113848 discloses an artificial respiration system comprising a helmet for non-invasive ventilation and a nasal cannula sampling respiratory airflow from a patient.

It is also known to use nasal cannulae which are generally provided with nozzles, for administering a breathable gas (mixture of oxygen and air) directly into a patient's nose, and are connected to a breathable gas supply circuit.

Nasal cannulae are used, for example, for the application of wet oxygen therapy which consists in the administration of a breathable gas flow higher than what is required by the patient. A FiO₂ (fraction of inspired oxygen) in a high percentage is thus supplied to the patient.

Clinical studies have shown that some diseases can be combated more effectively by the combined application of the CPAP therapy and the wet oxygen therapy.

Therefore, a need found in the prior art is to satisfy the need for medical devices which allow benefiting from this dual combined and concurrent therapy in a convenient, safe and efficient manner.

An object of the present invention is to satisfy this need within the context of a simple, patient-safe, rational and cost-effective solution.

These objects are achieved by the features of the invention described in the independent claim. The dependent claims describe preferred and/or particularly advantageous aspects of the invention.

### DESCRIPTION OF THE INVENTION

In particular, an embodiment of the invention provides a system for artificial respiration, comprising:
- a helmet provided with a container body, in which a patient's head can be accommodated, provided with an open end, an elastically yielding collar which can be sealingly coupled to the patient's neck and adapted to close the open end of said container body, inflow means of at least a first breathable gas within a volume enclosed by the container body and by the collar connectable to a first supply circuit of the first breathable gas; and outflow means from which the gases exhaled by the patient come out of said volume; and
- a nasal cannula provided with a flexible tube at a first end whereof a nasal dispenser is fixed for the administration of a second breathable gas directly into a patient's nose and a flexible tube, in which at least one portion of the flexible tube and the nasal dispenser of the nasal cannula are arranged inside the volume enclosed by the container body and by the collar of the helmet.
wherein the helmet is provided with a through opening, such as made in the container body or in the collar (or in any other suitable place such as a rigid ring(s) or other), which is adapted to put the outside of the helmet in communication with said inner volume; at least one portion of the nasal cannula being sealingly connected to said through opening of the helmet.

This solution provides a system which combines the wet oxygen therapy with the CPAP therapy which defines a closed environment in continuous positive airway pressure for the patient's forced respiration, in which the nasal cannula for wet oxygen therapy is also arranged.

Therefore, with this solution it is possible to simultaneously carry out both the CPAP therapy and the wet oxygen therapy so as to counteract some respiratory diseases more effectively.

With this solution, the nasal cannula can be connected to its supply circuit of the second breathable gas, for example in an independent manner with respect to the first supply circuit.

According to a preferred aspect of the invention, the system may comprise a connection device sealingly connected to (the through opening of) the helmet and to the flexible tube of the nasal cannula for the connection of the flexible tube of the nasal cannula to a supply circuit of the second breathable gas arranged outside the volume of the helmet, for example independent from the first supply circuit or in some way interconnected thereto.

With this solution, the system can, in a rational and convenient way for the user and the patient, make the helmet and the nasal cannula assembled for the efficient and convenient use of the dual therapy provided by the respiration system itself.

According to one aspect of the invention, the connection device can be connected to the nasal cannula in a removable manner.

With this solution, it is possible to adapt the connection device and, therefore, the helmet, to any nasal cannula, for example among those available on the market for wet oxygen therapy.

According to a further aspect of the invention, the connection device can be connected to the through opening in a removable manner.

With this solution, the nasal cannula can be previously installed in the proximity of the patient's nose and subsequently connected to the helmet, with greater convenience for the personnel in charge of the system installation on the patient and for the patient him/herself.

According to yet one aspect of the invention, the connection device may be connected to a second end of the flexible tube distal from the first end provided with the nasal dispenser.

Advantageously, the nasal cannula is all contained within the volume enclosed by the container body and by the collar of the helmet.

With this solution, the nasal cannula is permanently connected to the helmet and the sealed connection between them is particularly efficient and safe. According to a further aspect of the invention, the connection device is adapted to be removably connected to the second supply circuit extending, in fluid connection, the second end of the flexible tube of the nasal cannula from the interior towards the exterior of the volume enclosed by the container body and by the collar of the helmet.

With this solution, the connection between the nasal cannula and the second supply circuit, with the interposition of the connection device, is particularly effective and simple for the user and safe for the patient and for the success of the combined therapy.

According to one aspect of the invention, the connection device may comprise:
- a first fitting adapted to be sealingly connected to the through opening of the helmet;
- a second tubular fitting adapted to be arranged within the volume of the helmet (i.e. one free end whereof is adapted to be facing towards the interior of the helmet) and which is adapted to be sealingly connected to the flexible tube of the nasal cannula, preferably to the second end of the same; and
- a third tubular fitting adapted to be arranged outside the volume of the helmet (i.e. one free end whereof is adapted to be facing towards the exterior of the helmet) and which is adapted to be sealingly connected to the second supply circuit.

With this solution, by means of the same connection device it is possible to interface the various system components in a convenient, compact and rational manner.

Advantageously, the third fitting is of male (or female) type equal to a male (or female) connector of which the second end of the flexible tube of the nasal cannula is provided and the second fitting is of female (or male) type and it is adapted to mate with the male (or female) fitting of which the second end of the flexible tube of the nasal cannula is provided.

According to one aspect of the invention, the container body of the helmet can be provided with a rigid flange which delimits the through opening; and the first fitting may comprise a fixing ring sealingly fastened, for example by means of a threaded or bayonet or coupling (snap) connection or the like, to the rigid flange of the container body of the helmet.

With this solution the engagement of the connection device to the helmet is made simple, safe and quick.

A further aspect of the invention contemplates that the connection device may comprise a housing pocket adapted to house the second end of the flexible tube (which generally has the male or female connector), which housing pocket geometrically projects outside of the volume delimited by the container body of the helmet, or determines an appendage or a projection thereof facing (for example radially) outwards, and delimits a peripheral portion of the inner (fluid dynamic) volume, or the continuous positive airway pressure environment in which the patient's peripheral respiratory tract is inserted, enclosed by the container body and by the collar where the patient's head can be accommodated.

With this solution, the footprint of the second end of the flexible tube, or of the male connector fixed thereto, does not interfere with the inner space of the helmet in which the patient's head is accommodated and does not disturb or contact the patient him/herself.

According to one aspect of the invention, the flexible tube may be at least partially permeable for a water vapor contained in a second breathable gas.

With this solution, the water vapor transported by the nasal cannula, together with the oxygen, towards the patient's nose is also partially released, through the flexible tube, inside the continuous positive pressure volume enclosed by the container body and by the collar of the helmet, thereby allowing a synergy between the two respiratory therapies administered by means of the system.

Advantageously, the first breathable gas may be a dry mixture of air and oxygen and the second breathable gas may be a wet mixture of water vapor and oxygen and/or air.

Still another aspect of the invention contemplates that the second breathable gas may be a hot fluid at a temperature of between 30 °C and 40 °C.

For example, the second breathable gas may be supplied to the patient, by means of the nasal cannula, at a flow rate of between 1 l/min and 80 I/min.

### BRIEF DESCRIPTION OF THE DRAWINGS

Further features and advantages of the invention will become apparent from the following description, provided by way of non-limiting example with the aid of the figures shown in the accompanying drawings.
Figure 1 is an isometric view of the system for artificial respiration according to a preferred embodiment.
Figure 2 is an exploded view of a connection device of the system for artificial respiration in figure 1.

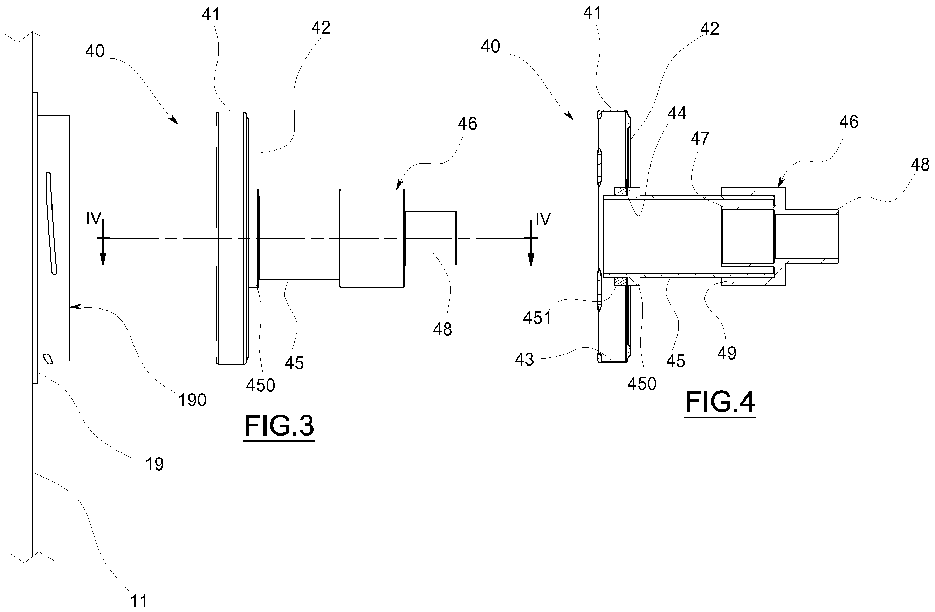

Figure 3 is a lateral view of the assembled connection device.
Figure 4 is the section IV-IV of figure 3.
Figure 5 is a lateral view of the connection device assembled to a nasal cannula of the respiration system.
Figure 6 is the section VI-VI of figure 5.

### BEST MODE OF CARRYING OUT THE INVENTION

With particular reference to such figures, reference numeral 100 globally indicates a system for artificial respiration which comprises a helmet 10 for non-invasive ventilation of patients in general.

Helmet 10 comprises a container body 11 which, advantageously, consists of a substantially cylindrical element, closed at one (upper) end and open, at the opposite end, of optically transparent material which, while being flexible, is not expansible.

Helmet 10 further comprises an elastically yielding collar 12 which can be fitted on the patient's neck and is adapted to close the open end of the container body 11.

Collar 12 is advantageously made of elastically yielding material to be sealingly coupled to the patient's neck.

Collar 12, in practice, has a substantially frustoconical shape whose upper open end of larger dimensions has a diameter substantially equal to the diameter of the lower open end of the container body 11 to which it is (removably or permanently) fixed, while the smaller (free) lower end, also open, has a diameter comparable to or slightly smaller than the diameter of the patient's neck.

Helmet 10 may be of closed or openable type, the features of an openable helmet are described hereinafter by way of non-limiting example. However, the invention may equally comprise a closed type helmet.

In closed type helmets, not shown in the figures, the container body 11 at the open lower end thereof is closed by a collar 12.

In a first variant of helmet 10 of the closed type, collar 12 may be fixed directly to the container body 11 by means of fastening techniques such as heat welding or other technique. For example, in this case, the helmet has no rigid structural parts such as rings or others that would prevent its folding in narrow spaces.

In a second variant of helmet 10 of the closed type, a rigid ring may be interposed between collar 12 and the container body 11, which for example keeps the circular shape of helmet 10 also when not in use.

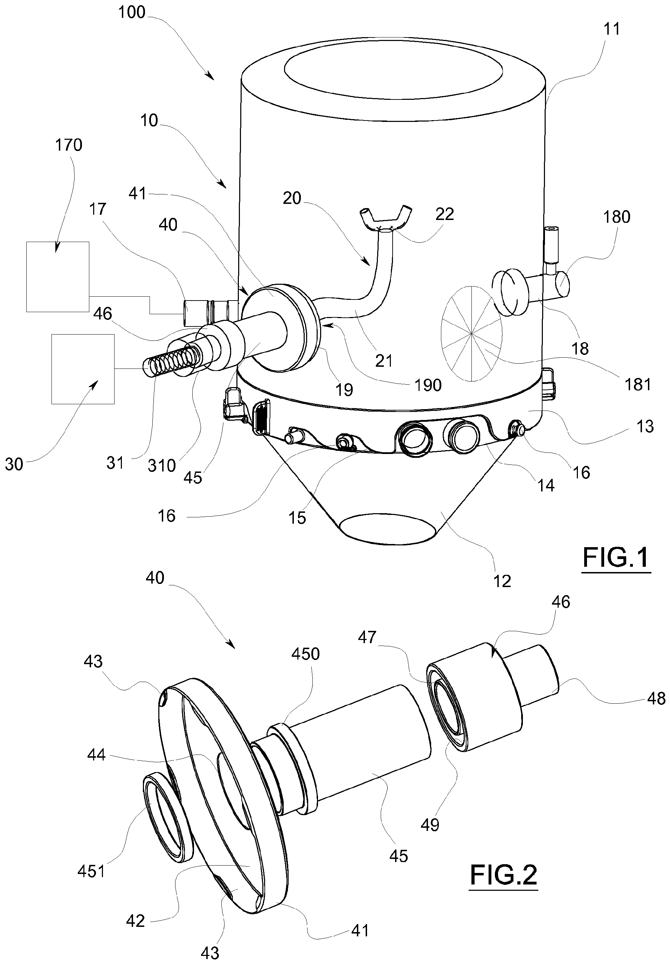

In openable helmets 10, a preferred embodiment of which is shown in figure 1, the container body 11 at the open lower end thereof is connected to a first rigid ring 13, by heat sealing or other fastening technique which ensures the hermetic and stable seal between them.

Collar 12 is connected to a second rigid ring 14, for example by means of fastening techniques which ensure the hermetic seal between collar 12 and the second rigid ring 14 itself.

The second rigid ring 14, which may for example be coated with soft material, may be sealingly associated, so as to be removable, to the first rigid ring 13 (which may also for example be coated with soft material), as will become apparent in the following description.

Helmet 10 may also comprise an annular gasket compressed between the rigid rings 13 and 14 when helmet 10 is in the assembled closed configuration.

The openable helmet 10 comprises removable locking means adapted to removably reciprocally lock the first rigid ring 13 and the second rigid ring 14, which for example are selected from the group including bayonet couplings, snap couplings and threaded couplings.

Only the preferred embodiment shown in the figures is described hereinafter, in which the rigid rings 13 and 14 are provided with locking means of the bayonet coupling type which comprise two coupling elements 15 and 16 which are associated to the first rigid ring 13 and to the second rigid ring 14, respectively.

The coupling elements, in particular, comprise a plurality of pins 16, for example fixed to a lateral surface of the second rigid ring 14, and a respective plurality of housing seats 15, for example formed in the first rigid ring 14.

In practice, each pin 16 is adapted to be removably inserted in a housing seat 25 following a contained reciprocal translation-rotation between the first and the second rigid with respect to the mutual approach axis.

In the embodiment shown in the figures, the second rigid ring 14 is adapted to slip substantially snugly inside the first rigid ring 13, in a coaxial manner; the bayonet coupling means comprise a plurality of pins 16, which protrude radially from the outer lateral surface of the second rigid ring 14 and, for example, are mutually substantially equidistant, and a corresponding plurality of housing seats 25, formed in the first rigid ring 13 and, for example, are also mutually equidistant.

Some embodiments of helmet 10 may contemplate that the container body 11 is provided with openings closable by means of a hinge (or other) to form an access for the patient.

Helmet 10 (either of openable type or closed type) further comprises inflow means of a first breathable gas, for example air (or a mixture of air and oxygen or oxygen), within the inner volume enclosed by the container body 11 and by collar 12 and outflow means of the gases exhaled by the patient during breathing from the inner volume itself.

In the example shown, the inflow means comprise an inlet duct 17 for the air supply (for example, of standard size for the connection to usual breathable gas supply tubes such as air, oxygen or air and oxygen, venturimeters or similar devices), which is for example provided, at the end flowing inside the volume enclosed by the container body 11 and by collar 12, with a diffuser.

The inlet duct 17 is for example connected, by means of said tube, to a first supply circuit 170 of such a first breathable gas, for example at suitably adjustable and calibrated flows and concentrations depending on the therapy to be administered.

The outflow means comprise an outlet duct 18 of the gases exhaled by the patient in breathing (for example, of standard size for the connection to usual tubes or PEEP valves or other similar devices), which for example is also provided, at the end flowing inside the volume enclosed by the container body 11 and by collar 12, with a diffuser.

Helmet 10 comprises a PEEP valve 180, (removable or permanently) fixed to the outlet duct 18, through which the gases exhaled by the patient in breathing exit, which thus defines the above outflow means and ensures a certain (adjustable) positive pressure inside the inner volume enclosed by the container body 11 and by collar 12.

In the example shown, the inlet duct 17 and the outlet duct 18 are both fixed to the container body 11, for example, in any part of the same.

An anti-suffocation valve 181 may also be fixed on the container body, i.e. a bi-directional valve that is able to put the exterior in communication with the interior of the container body 11 in an emergency, or when a pressure drop below a settable threshold value occurs inside the volume enclosed by the container body 11 and by helmet 12. For example, the anti-suffocation valve 181 may be of the bi-directional type of the type described in patent EP 1 797 925 to the name of the same Applicant.

It is not excluded, however, that the anti-suffocation valve 181 may be positioned on a further duct provided for that purpose in helmet 10 and placed in any desired position.

Moreover, on the same helmet 10 there may be provided more than one inlet duct 17 and more than one outlet duct 18 depending on the construction or therapy requirements for the patient.

It is not excluded, moreover, that at least one of the inlet 17 and outlet 18 ducts may have a curved axis, unlike those shown, which have a straight (and radial) axis.

Helmet 10 may further comprise retaining means adapted to exert a pulling action of helmet 10 towards the patient's shoulders with the function of preventing the raising of helmet 10 due to the positive pressure inside the same.

Such retaining means may be of the "axillary strap" or "rigid board" or "tie rod" type, as known to the man skilled in the art, or of different type or according to the requirements.

Helmet 10 further comprises a through opening 190, for example formed at the container body 11, or at a front portion thereof (facing the patient's face). The through opening 190 for example is substantially circular and has such a size (diameter) as to be crossed by a man's hand (an operator).

The through opening 190 is delimited by a rigid flange 19, for example annular in shape, which for example has a substantially cylindrical tang protruding towards the outside of the container body 11.

The rigid flange 19 is fixed to the container body 11, for example by means of heat sealing or other suitable fixing means.

In particular, the rigid flange 19 (i.e. its internal bore) has a shape and size substantially homologous to those of the through opening 190, i.e., it is wide enough to allow an operator to insert at least one hand inside the container body 11, allowing a suitable maneuvering space on the patient wearing helmet 10.

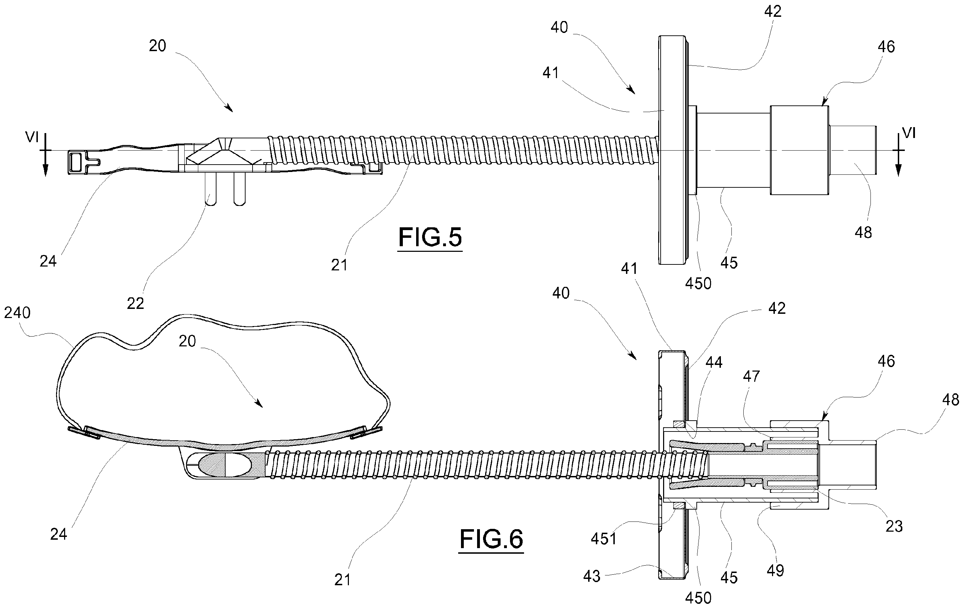

System 100 further comprises a nasal cannula 20, shown in detail in figures 5 and 6.

The nasal cannula 20 is a tube configured to administer a second breathable gas directly into the patient's nose when this wears helmet 10 described above.

In particular, the nasal cannula 20 is configured for the application of wet oxygen therapy.

The nasal cannula 20 comprises a flexible tube 21.

The flexible tube 21 is for example of the corrugated tube type.

The flexible tube 21 may for example be made of a permeable, or at least partially permeable, material with respect to water vapor.

In practice, the flexible tube 21 is adapted to emit toward the outside of the flexible tube 21 a quantity of water vapor carried within it, such as water vapor contained in the second breathable gas.

The flexible tube 21 has a length substantially comparable to the perimeter of a patient's head circumference.

The flexible tube 21 is provided with a first end to which a nasal dispenser 22 is associated, such as permanently attached or removable.

The nasal dispenser 22, for example provided with one or two dispensing nozzles, is adapted to dispense the second breathable gas directly into the patient's nostrils, namely the patient whose head is placed inside the volume enclosed by the container body 11 and by collar 12 of helmet 10.

Each nozzle is configured not to occlude more than 50% of the inner diameter of the patient's single nostril.

Moreover, each nozzle must be wide enough not to compress the patient's nasal septum.

A single nozzle may for example be used for the therapy of newborns.

For example, the outer diameter of each nozzle is comprised between 1.5 mm and 4.8 mm, in which for newborns and infants, the outer diameter is comprised between 1.5 mm and 1.9 mm, for pediatric patients the outer diameter is comprised between 1.9 mm and 2.7 mm and for adult patients, the outer diameter is comprised between 2.7 mm and 4.8 mm, such as substantially equal to 4.8 mm.

The flexible tube 21 is then provided with a second free end (opposite to the first end).

The second end is for example associated (such as permanently or removably attached) to a connector 23, such as a standard (22M) male (tubular) connector.

Connector 23 is for example configured to connect the nasal cannula 20 with a second supply circuit 30 of the second breathable gas.

For example, the second supply circuit 30 comprises a supply group of the second breathable gas provided with elements for adjusting at least one of the following parameters among flow, pressure, temperature, concentration and composition of the second breathable gas itself.

The second breathable gas for example is a mixture of air, oxygen and water vapor or oxygen and water vapor or other suitable mixture, for example the second breathable gas may also not be moist or not have water vapor.

The second breathable gas may also comprise helium (He), i.e. to be a Helium and Oxigen (and water vapeor) mixture, which - having a lower density than air - reduces the breathing work by reducing the force required to transfer the gas through the airways.

The second breathable gas may be a hot fluid at a temperature comprised between 30 °C and 40 °C, for example between 36° and 37 °C.

For example, the second breathable gas may be fed (by the second supply circuit 30) at a flow rate comprised between 1 l/min and 80 l/min, for example between 1 l/min and 8 l/min (low flow) or 5-40 It/min (high flow).

The second supply circuit 30 ends with a dispenser tube 31, whose downstream end (in the flowing direction of the second breathable gas supplied) is provided with a suitable connector, such as for instance a male connector 310.

For example, the first supply circuit 170 and the second supply circuit 30 are independent of each other, but may be interconnected by a suitable fitting, such as a "Y" fitting, provided with an inlet branch and two outlet branches, in which the inlet branch is associated with a source of breathable gas (such as oxygen and/or air), one of the two outlet branches is intended to be connected to the nasal cannula (20) and the other outlet branch to helmet 10 (to the inlet duct 17 thereof). For example, the outlet branch connected to the nasal cannula is associated to a humidifier/vaporizer for the humidification of the second breathable gas introduced into the nasal cannula 20. The nasal cannula 20 further comprises a group for anchoring the nasal dispenser 22 to the patient's face.

Preferably, the anchoring group comprises, for example, a pair of strings 24 attached to the first end of the flexible tube 21 and branching off from opposite sides with respect to the nasal dispenser 22.

Each of strings 24 may then be fixed to a respective portion of a band 240, such as elastic, adapted to be fitted on the patient's head for retaining the nasal dispenser 22 in the vicinity of the nose of the patient him/herself, without engaging the mouth and the eyes of the patient him/herself. Connector 23 is configured to connect to the female connector 310, as will be better described hereinafter, so as to connect the nasal cannula 20 to the second supply circuit 30 for the supply of the second breathable gas directly into the patient's nose.

System 100 further comprises a connection device 40 configured to interconnect helmet 10 and the nasal cannula 20, for example by ensuring the insulation (without air leaks) between the volume enclosed within the container body 11 and of collar 12 of helmet 10 and the volume outside helmet 10.

For example, the connection device 40 is also configured to interconnect the nasal cannula 20 with the second supply circuit 30, as will be better shown hereinafter.

The connection device 40, in fact, consists of a multiple fittings assembly, as will be better described hereinafter in the description.

The connection device 40 is removably fixed to helmet 10, preferably to the container body 11 of helmet 10, at the through opening 190.

In practice, the connection device 40 is sealingly connected to the through opening 190, for example by means of a first connection fitting.

The connection device 40, in particular, comprises a fixing ring 41, such as circular and having a shape homologous to the shape of the rigid flange 19 surrounding the through opening 190, which defines (and constitutes) said first connection fitting.

The fixing ring 41 for example comprises a cylindrical shank, adapted to be fitted on the cylindrical shank of the rigid flange 19 and define a removable stable coupling therewith.

For example, threaded or bayonet or snap fitting or interference connections or the like are defined between the cylindrical shank of the rigid flange 19 and the cylindrical shank of the fixing ring 41.

The fixing ring 41 for example is of a cover or porthole type.

The fixing ring 41 for example comprises a sealing element, such as an annular gasket, which is adapted to be compressed (axially or radially) between the fixing ring 41 and the cylindrical shank of the rigid flange 19.

In the embodiment shown in the figures, the fixing ring 41 comprises a disc-shaped body 42, whose outer diameter is substantially equal to or slightly larger than the diameter of the through opening 190.

The disc-shaped body 42 is provided with a folded edge 43 (or shank) which is associated to a plurality of mutually equidistant fixing tabs which protrude towards the interior of the folded edge 43 and which define, with respective male thread portions defined on the cylindrical shank of the rigid flange 19, the above threaded connection.

The fixing ring 41 further comprises a through hole 44 made in the disc-shaped body 42 and, for example, coaxial thereto.

The through hole 44 has a diameter substantially greater than the maximum diameter of the flexible tube 21 (and of connector 23) of the nasal cannula 20, such as a diameter greater than the maximum diameter of connector 23. The connection device 40 further comprises a tubular sleeve 45, for example having substantially cylindrical and hollow shape, which is for example fixed to the fixing ring 41 and originates from the disc-shaped body 42 of the fixing ring 41 on the side opposite to the folded edge 43 of the same.

The tubular sleeve 45 is fixed to the fixing ring 41 coaxially to the through hole 44, actually extending the latter.

The inner diameter of the tubular sleeve 45 is substantially equal to or slightly smaller than the diameter of the through hole 44 and, anyway, is greater than the maximum diameter of the flexible tube 21 (and of connector 23) of the nasal cannula 20 (that is, greater than the maximum diameter of connector 23).

The tubular sleeve 45 is provided with an end, proximal to the fixing ring 41, which is constrained to the disc-shaped body 42 of the fixing ring itself and to an opposite free end.

For example, the longitudinal axis of the tubular sleeve 45 is substantially perpendicular to the lying plane of the disc-shaped body 42 of the fixing ring 41 (such as parallel and coincident with the screwing axis of the fixing ring 41 on the rigid flange 19).

In practice, the tubular sleeve 45 geometrically projects (for example in the radial direction) towards the exterior of the container body 11 of helmet 10 when the fixing ring 41 is anchored to the rigid flange 19.

The tubular sleeve 45, in the example, is removably fixed to the disc-shaped body 42 of the fixing ring 41.

For example, the constrained end of the tubular sleeve 45 is fitted with reduced radial clearance or with interference into the through hole 44.

In any case, a fluid-tight connection is defined between the tubular sleeve 45 and the fixing ring 41 (or the disc-shaped body 42 of the same).

The tubular sleeve 45 comprises, for example, a radial projection 450 (whose outer diameter is greater than the diameter of the through hole 44) formed in the vicinity of the constrained end and adapted to act as a travel end element of the axial sliding of the tubular sleeve 45 (in a first sliding direction) with respect to the disc-shaped body 42 of the fixing ring 41.

The connection device 40, in this case, further comprises a locking ring 451 (whose outer diameter is greater than the diameter of the through hole 44) adapted to fit snugly over the constrained end of the tubular sleeve 45.

The locking ring 451 actually constitutes an anti-extraction element (in the second sliding direction opposite to the first one) for the tubular sleeve 45.

In practice, the disc-shaped body 42 of the fixing ring 41 is located, once fitted with its through hole 44 on the constrained end of the tubular sleeve 45, axially interposed (and fixed) between the locking ring 451 and the radial projection 451 which prevent axial sliding of the tubular sleeve 45 with respect to the disc-shaped body 42 of the fixing ring 41.

The locking ring 451 may be connected to the tubular sleeve 45 by known fixing means, such as threaded fasteners, snap or bayonet couplings or the like.

The tubular sleeve 45 may alternatively be made in a single body with the fixing ring 41, or with the disc-shaped body 42 thereof.

The tubular sleeve 45 (or a large portion of the same provided with the free end thereof) is actually geometrically placed outside the geometrical volume of helmet 10 (or of the container body 11) when it is fixed to the fixing ring 41 and when this is fixed to the rigid flange 19.

The tubular sleeve 45 is fixed to the fixing ring 41 and when this is fixed to the rigid flange 19, it defines a housing pocket geometrically projecting outside the volume delimited by the container body 11 of helmet 10 and delimits, in its inner cavity, a peripheral portion (an extension) of the inner fluid volume enclosed by the container body 11 and by collar 12 where the patient's head can be accommodated. In practice, the inner volume of the tubular sleeve 45 is in fluid communication with the inner volume enclosed by the container body 11 and by collar 12 and delimits a small additional peripheral portion thereof communicating with it.

The axial length of the tubular sleeve 45 is for example, comparable (equal to or slightly greater or slightly smaller than) the axial length of connector 23 of the nasal cannula 20.

In practice, connector 23 can be accommodated substantially to measure or with good clearance inside the housing pocket defined by the tubular sleeve 45, as will be better described hereinafter.

The connection device 40 further comprises a connector body 46 fixed to the fixing ring 41, as will be better described hereinafter, and configured to sealingly interconnect (in fluid communication) the nasal cannula 20 with the tubular sleeve 45 and thus with the fixing ring 41.

In the example shown, the connector body 46 is configured to sealingly interconnect (in fluid communication) the nasal cannula 20 to the second supply circuit 30, or to the female connector 310 thereof.

In this case, the connector body 46 is a three-fitting connector, i.e. is adapted to be connected to three different elements of system 100 for the mechanical and/or fluid dynamic interconnection of the same.

The connector body 46 comprises a central tubular body provided with an inner cavity open at the opposite axial ends.

At a first end, the central tubular body has a tubular second fitting 47, such as of female type, preferably of standard 22F size, which is adapted to be sealingly connected (by interference or other types of known and/or standard connections or couplings) to connector 23 of the nasal cannula 20.

In addition, the central tubular body has a third fitting 48 at the opposite end, such as of male type, preferably of standard 22M size, which is adapted to be sealingly connected (by interference), for example, to the female connector 310 of the dispenser tube 31 of the second supply circuit 30.

In practice, the second fitting 47 is complementary to connector 23 of the nasal cannula 20 and the third fitting 48 is identical to connector 23 of the nasal cannula 20. The central tubular body of the connector body 46 is actually a (rigid) extension of connector 23 of the nasal cannula 20. Advantageously, the second fitting 47 of the connector body 46 is configured to axially retain connector 23 of the nasal cannula 20, for example by interference or snap couplings or bayonet couplings.

Advantageously, the third fitting 48 of the connector body 46 is configured to axially retain the female connector 31 of the dispenser tube 31 of the supply circuit 30, for example by interference or snap couplings or bayonet couplings or other known coupling types.

The connector body 46 further comprises fixing elements to the fixing ring 41, such as to the tubular sleeve 45.

In detail, the connector body 46 also comprises a peripheral tubular bodywhich (coaxially) surrounds at a distance at least an axial portion of the central tubular body and is sealingly attached thereto by a closing ring.

In practice, the closing ring is axially interposed between the first end and the second end of the central tubular body, i.e. axially divides the second fitting 47 and the third fitting 48.

The peripheral tubular body is placed outside in circumferential (and coaxial) direction (only) to the second fitting 47 of the central tubular body (leaving the entire third fitting 48 exposed in circumferential direction).

In practice, the second fitting 47 is inserted with plenty of radial clearance, i.e. with a radial clearance greater than the thickness of the tubular sleeve 45, inside the peripheral tubular body.

The peripheral tubular body, or at least the free end thereof (aligned or staggered with respect to the free end of the second fitting 47) defines a tubular fourth fitting 49, for example female, which is adapted to be sealingly connected (by interference), for example, to the free end of the tubular sleeve 45.

For example, the fourth fitting 49 may be connected to the free end of the tubular sleeve 45 through couplings or connections, such as snap couplings, bayonet couplings, threaded connections and the like.

In practice, the free end of the tubular sleeve 45 may be inserted by interference within the fourth fitting 49, while fitting the second fitting 47 with plenty of radial clearance.

It is not excluded that the fourth fitting 49 may be male adapted to be inserted inside the free end of the tubular sleeve 45.

As an alternative to the simple sealed interference shown in the figures, the fourth fitting 49 may comprise sealed coupling means adapted to couple to suitable complementary means present on the tubular sleeve 45, for example the coupling means may be bayonet or snap-type or other.

The second fitting 47, as shown and described, once the connector body 46 is fixed to the tubular sleeve 45, is located within the fluid volume enclosed by the container body 11, by collar 12 and by the connection device 40, when assembled, i.e. located in the patient's breathing environment.

In this example, the connector body 46 is made in a separate body with respect to the fixing ring 41 and to the tubular sleeve 45, but it is not excluded that it may be made in a single body with one of them or with both. The connector body 46 actually occludes (except for the through opening defined by the axial cavity of the central tubular body) the free end of the tubular sleeve 45 and thud the through opening 190 of helmet 10.

When system 100 is in use, the nasal cannula 20 is intended to be contained (completely, in the example) within the volume enclosed by the container body 11, by collar 12 and by the connection device 40, i.e. the patient's breathing environment set at constant positive pressure by the first supply circuit 170 and by the outflow means.

In practice, the nasal cannula 20, as shown in figures 5 and 6, is connected to the connection device 40 by means of its second end, and the connection device 40 is connected to helmet 10 and to the second supply circuit 30.

In this case, the entire flexible tube 21 is placed within the volume enclosed by the container body 11 and by collar 12 of helmet 10, also allowing a controlled humidification of the patient's breathing environment (at continuous positive pressure), through the transpiration of the water vapor therethrough.

It is not excluded that the nasal cannula 20 may be only partially inserted inside helmet 10 and that it, therefore, exits through the connection device 40 (which will define a sealed connection with an axial portion thereof different from the second end). In this case, the nasal cannula 20 may be connected to the second supply circuit 30 directly, while the connection device 40 will be connected to helmet 10 and, as mentioned, to the axial portion of the nasal cannula 20.

To set system 100 on the patient's head, it is possible to proceed as described hereinafter.

The nasal cannula 20 may be first associated to the patient's head by inserting or approaching the nasal dispenser 21 to the nostrils of the same and wrapping strings 24 and band 240 behind the patient's neck.

The second end of the nasal cannula 20 in this step has connector 23 free, i.e. disconnected from anything.

Once the nasal dispenser 22 has been fitted, helmet 10 may be fitted on the patient's head, such as by enlarging the opening of collar 12, fitting helmet 10 from above and releasing collar 12 in the vicinity of the neck, where it adhere3s by exerting a a fluid-dynamic seal.

The nasal cannula 20, i.e. the second end thereof, will thus lie within the volume enclosed by helmet 10, such as resting on the inner surface of collar 12.

The personnel in charge of the setting, having previously removed the connection device 40 from helmet 10, now fits the second end of the nasal cannula 10 in the through opening 19, leading connector 23 outside of the inner volume of helmet 10 for convenience of work.

Thereafter, connector 23 may be stably connected to the second fitting 47 (for example having first connected or providing then to connect the fourth fitting 49 to the free end of the tubular sleeve 45, in turn connected to the fixing ring 41).

Connector 23 therefore actually occupies (with clearance) the housing pocket defined within the tubular sleeve 45 and does not interfere with the patient's head housing space, accommodated inside helmet 10, which will thus be clear of obstacles and with no parts in contact with the patient's face.

When the connection device 40 is thus connected to connector 23 of the nasal cannula 20 (see figures 5 and 6) and the nasal cannula 20 is fixed to the patient's head as described above, the operator can proceed to fasten the connection device 40 to helmet 10.

In practice, by inserting the nasal cannula 20 again into the inner volume of helmet 10, the locking ring 10 is connected (by screwing) to the rigid flange 19, thereby actually occluding the through opening 190.

In this configuration, system 100 is assembled and the nasal cannula 20 is entirely contained within the container body 11 of helmet 10.

Therefore, the operator can complete the setting of system 100 for administering the (combined) therapy by connecting the second supply circuit 30 of the second breathable gas to the nasal cannula 20, that is, by connecting the female connector 310 to the third fitting 38 of the connector body 46 of the connection device 40, and connecting the first supply circuit 170 to the inlet duct 17 having placed, for example on the outlet duct 18, the PEEP valve 180 suitably regulated.

The supply of first breathable gas through the inlet duct 17 allows obtaining a continuous positive pressure for the CPAP therapy inside the volume enclosed by the container body 11 and by collar 11 of helmet 10, while the supply of second breathable (wet) gas through the connection device 40 and the nasal cannula 20 allows having a direct oxygenation of the patient's airway (simultaneously with the CPAP) and a humidification of the patient's own breathing environment, i.e. the positive pressure environment inside helmet 10, through the transpiration of water vapor through the flexible tube 21. Alternative embodiments may provide that the through opening 190 is made in collar 12 so that the nasal cannula 20 passes therethrough.

For example, the through opening 190 may be the same main opening with which collar 12 is worn on the patient's neck (the main opening that allows the head to be inserted into collar 12) or be an extension thereof (for example for a limited radial section), or be defined by a slot communicating with the main opening defined, for example, in the vicinity of a lateral portion of collar 12.

In these embodiments, the elastic deformability of collar 12 itself is such that the connection between collar 12 (or the through opening 190) and the portion of nasal cannula 20 (for example of flexible tube 21) fitted therein is sealed, such as by elastically (forced) retaining said portion of nasal cannula 20 between the patient's neck and a lip of collar 12 surrounding the through opening 190.

In other embodiments, the through opening 190 may be a different through opening made in collar 12 and different from the above main opening.

For example, the through opening 190 in this case may be defined by an eccentric hole of collar 12, for example surrounded by a suitable flange and a suitable fitting, of suitable size to allow the passage of at least one end portion of the nasal cannula 20.

In this case, the seal between the portion of nasal cannula 20 fitted into the through opening 190 and collar 12 may be achieved using the elastic deformability of the same material with which collar 12 is made, for example when the through opening 190 is extendable elastically or through suitable pre-drilled or drillable baffles, for example fixed to enclose said suitable flange/fitting.

Several changes and variations may be made to the invention thus conceived, all falling within the scope of the inventive concept.

Moreover, all details can be replaced with other technically equivalent elements.

In practice, the materials used as well as the shapes and sizes may be any according to the requirements, without departing from the protection scope of the following claims.

## Claims

1. An artificial respiration system (100), comprising:
- a helmet (10) provided with a container body (11), in which a patient's head can be accommodated, provided with an open end, a resiliently yielding collar (12) which can be sealingly coupled to the patient's neck and adapted to close the open end of said container body (11), inflow means (17) of at least a first breathable gas within a volume enclosed by the container body (11) and by the collar (12), wherein the inflow means (17) are connectable to a first supply circuit (170) of the first breathable gas; and outflow means (18) from which the gases exhaled by the patient come out of said volume;
- a nasal cannula (20) provided with a flexible tube (21) at a first end whereof a nasal dispenser (22) is fixed for the administration of a second breathable gas directly into a patient's nose, wherein at least one portion of the flexible tube (21) and the nasal dispenser (22) of the nasal cannula (20) are arranged inside the volume enclosed by the container body (11) and by the collar (12) of the helmet (10),
**characterized in that** the helmet (10) is provided with a through opening (190) adapted to put the outside of the helmet (10) in communication with said inner volume; at least one portion of the nasal cannula (20) being sealingly connected to said through opening (190) of the helmet (10).

2. System (100) according to claim 1, which comprises a connection device (40) sealingly connected to the through opening (190) of the helmet (10) and to the flexible tube (21) of the nasal cannula (20) for connecting the flexible tube (21) of the nasal cannula (20) to a second supply circuit (30) of the second breathable gas arranged outside the volume of the helmet (30).

3. System (100) according to claim 2, wherein the connection device (40) is disengageably connected to the nasal cannula (20).

4. System (100) according to any one of the preceding claims 2-3, wherein the connection device (40) is disengageably connected to the through opening (190).

5. System according to any one of the preceding claims 2-4, wherein the connection device (40) is connected to a second end of the flexible tube (21) distal from the first end provided with the nasal dispenser (22).

6. System according to claim 5, wherein the nasal cannula (20) is totally contained within the volume enclosed by the container body (11) and by the collar (12) of the helmet (10).

7. System according to claim 5 or 6, wherein the connection device (40) is adapted to be disengageably connected to the second supply circuit (30) extending the second end of the flexible tube (21) of the nasal cannula (20) towards the outside of the volume enclosed by the container body (11) and by the collar (12) of the helmet (10).

8. System (100) according to any one of the preceding claims 2-7, wherein the connection device (40) comprises:
- a first fitting (41) adapted to be sealingly connected to the through opening (190) of the helmet (10);
- a tubular second fitting (47) adapted to be arranged within the fluid volume enclosed by the container body (11), by the collar (12) of the helmet (10) and by connection device (40), when assembled, and which is adapted to be sealingly connected to the flexible tube (21) of the nasal cannula (20), preferably to the second end of the same; and
- a tubular third fitting (48) adapted to be arranged outside the volume of the helmet (10) and which is adapted to be sealingly connected to the second supply circuit (30).

9. System (100) according to claim 8, wherein the third fitting (48) is of male type equal to a male connector (23) the second end of the flexible tube (21) of the nasal cannula (20) is provided with, and the second fitting (47) is of female type and is adapted to mate with the male connector (23) the second end of the flexible tube (21) of the nasal cannula (20) is provided with.

10. System (100) according to claim 8 or 9, wherein:
- the container body (11) of the helmet (10) is provided with a rigid flange (19) which delimits the through opening (190); and wherein
- the first fitting is defined by a fixing ring (41) sealingly fastened to the rigid flange (19) of the container body (11) of the helmet (10).

11. System (100) according to claim 5, wherein the connection device (40) comprises a housing pocket (45) adapted to house the second end of the flexible tube (21), which housing pocket (45) geometrically projects outside the volume delimited by the container body (11) of the helmet (10) and delimits a peripheral portion of the inner volume enclosed by the container body (11) and by the collar (12) where the patient's head can be accommodated.

12. System (100) according to claim 1, wherein the flexible tube (21) is at least partially permeable for a water vapor contained in the second breathable gas.

13. System according to claim 1, wherein the first breathable gas is a dry mixture of air and oxygen and the second breathable gas is a wet mixture of water vapor and oxygen and/or air.

14. System according to claim 1, wherein the second breathable gas is a hot fluid at a temperature comprised between 30 °C and 40 °C.

15. System according to claim 1, wherein the through opening (190) is formed in the container body (11).

16. System according to claim 1, wherein the through opening (190) is formed in the collar (12).

## Patentansprüche

1. System zur künstlichen Beatmung (100), das Folgendes umfasst:
- einen Helm (10), der mit einem Behälterkörper (11) versehen ist, in dem der Kopf eines Patienten untergebracht werden kann, der mit einem offenen Ende versehen ist, einen elastisch nachgiebigen Kragen (12), der abdichtend mit dem Hals des Patienten gekoppelt werden kann und dazu angepasst ist, das offene Ende des Behälterkörpers (11) zu verschließen, Einströmmittel (17) für mindestens ein erstes atembares Gas innerhalb eines Volumens, das von dem Behälterkörper (11) und dem Kragen (12) umschlossen ist, wobei die Einströmmittel (17) mit einem ersten Zuführkreislauf (170) des ersten atembaren Gases verbindbar sind; und Ausströmmittel (18), aus denen die vom Patienten ausgeatmeten Gase aus diesem Volumen austreten;
- eine Nasenkanüle (20), die mit einem flexiblen Schlauch (21) versehen ist, an dessen erstem Ende eine nasale Abgabeeinrichtung (22) zur Verabreichung eines zweiten atembaren Gases direkt in die Nase eines Patienten fixiert ist, wobei mindestens ein Abschnitt des flexiblen Schlauchs (21) und der nasalen Abgabeeinrichtung (22) der Nasenkanüle (20) innerhalb des vom Behälterkörper (11) und vom Kragen (12) des Helms (10) umschlossenen Volumens angeordnet ist, **dadurch gekennzeichnet, dass** der Helm (10) mit einer Durchgangsöffnung (190) versehen ist, die dazu angepasst ist, die Außenseite des Helms (10) mit dem Innenvolumen in Verbindung zu bringen; wobei mindestens ein Abschnitt der Nasenkanüle (20) abdichtend mit der Durchgangsöffnung (190) des Helms (10) verbunden ist.

2. System (100) gemäß Anspruch 1, das eine abdichtend mit der Durchgangsöffnung (190) des Helms (10) und mit dem flexiblen Schlauch (21) der Nasenkanüle (20) verbundene Verbindungsvorrichtung (40) zur Verbindung des flexiblen Schlauchs (21) der Nasenkanüle (20) mit einem zweiten, außerhalb des Volumens des Helms (30) angeordneten Zuführkreislauf (30) für das zweite atembare Gas umfasst.

3. System (100) gemäß Anspruch 2, wobei die Verbindungsvorrichtung (40) lösbar mit der Nasenkanüle (20) verbunden ist.

4. System (100) gemäß einem der vorhergehenden Ansprüche 2-3, wobei die Verbindungsvorrichtung (40) lösbar mit der Durchgangsöffnung (190) verbunden ist.

5. System gemäß einem der vorhergehenden Ansprüche 2-4, wobei die Verbindungsvorrichtung (40) mit einem zweiten Ende des flexiblen Schlauchs (21) verbunden ist, das distal von dem ersten Ende liegt, das mit der nasalen Abgabeeinrichtung (22) versehen ist.

6. System gemäß Anspruch 5, wobei die Nasenkanüle (20) vollständig in dem vom Behälterkörper (11) und vom Kragen (12) des Helms (10) umschlossenen Volumen enthalten ist.

7. System gemäß Anspruch 5 oder 6, wobei die Verbindungsvorrichtung (40) zur lösbaren Verbindung mit dem zweiten Zuführkreislauf (30) angepasst ist, wobei das zweite Ende des flexiblen Schlauchs (21) der Nasenkanüle (20) zur Außenseite des vom Behälterkörper (11) und vom Kragen (12) des Helms (10) umschlossenen Volumens verlängert wird.

8. System (100) gemäß einem der vorhergehenden Ansprüche 2-7, wobei die Verbindungsvorrichtung (40) Folgendes umfasst:
- ein erstes Anschlussstück (41), das dazu angepasst ist, mit der Durchgangsöffnung (190) des Helms (10) abdichtend verbunden zu werden;
- ein rohrförmiges zweites Anschlussstück (47), das dazu angepasst ist, in dem vom Behälterkörper (11), vom Kragen (12) des Helms (10) und von der Verbindungsvorrichtung (40) umschlossenen Fluidvolumen angeordnet zu werden, wenn diese zusammengebaut sind, und das dazu angepasst ist, mit dem flexiblen Schlauch (21) der Nasenkanüle (20), vorzugsweise mit dem zweiten Ende derselben, abdichtend verbunden zu werden; und
- ein rohrförmiges drittes Anschlussstück (48), das dazu angepasst ist, außerhalb des Volumens des Helms (10) angeordnet zu werden und das dazu angepasst ist, abdichtend mit dem zweiten Zuführkreislauf (30) verbunden zu werden.

9. System (100) gemäß Anspruch 8, wobei das dritte Anschlussstück (48) vom Einstecktyp gleich einem Steckverbinder (23) ist, mit dem das zweite Ende des flexiblen Schlauchs (21) der Nasenkanüle (20) versehen ist, und wobei das zweite Anschlussstück (47) vom Aufnahmetyp ist und dazu angepasst ist, mit dem Steckverbinder (23) zusammenzupassen, mit dem das zweite Ende des flexiblen Schlauchs (21) der Nasenkanüle (20) versehen ist.

10. System (100) gemäß Anspruch 8 oder 9, wobei:
- der Behälterkörper (11) des Helms (10) mit einem starren Flansch (19) versehen ist, der die Durchgangsöffnung (190) begrenzt; und wobei
- das erste Anschlussstück durch einen Fixierring (41) definiert ist, der abdichtend am starren Flansch (19) des Behälterkörpers (11) des Helms (10) befestigt ist.

11. System (100) gemäß Anspruch 5, wobei die Verbindungsvorrichtung (40) eine Aufnahmetasche (45) umfasst, die dazu angepasst ist, das zweite Ende des flexiblen Schlauchs (21) aufzunehmen, wobei die Aufnahmetasche (45) geometrisch aus dem durch den Behälterkörper (11) des Helms (10) begrenzten Volumen herausragt und einen Umfangsabschnitt des durch den Behälterkörper (11) und durch den Kragen (12) umschlossenen Innenvolumens begrenzt, wo der Kopf des Patienten untergebracht werden kann.

12. System (100) gemäß Anspruch 1, wobei der flexible Schlauch (21) mindestens teilweise durchlässig für Wasserdampf ist, der in dem zweiten atembaren Gas enthalten ist.

13. System gemäß Anspruch 1, wobei das erste atembare Gas ein trockenes Gemisch aus Luft und Sauerstoff und das zweite atembare Gas ein feuchtes Gemisch aus Wasserdampf und Sauerstoff und/oder Luft ist.

14. System gemäß Anspruch 1, wobei das zweite atembare Gas ein warmes Fluid mit einer Temperatur, die zwischen 30 °C und 40 °C liegt.

15. System gemäß Anspruch 1, wobei die Durchgangsöffnung (190) in dem Behälterkörper (11) ausgebildet ist.

16. System gemäß Anspruch 1, wobei die Durchgangsöffnung (190) in dem Kragen (12) ausgebildet ist.

## Revendications

1. Système de respiration artificielle (100), comprenant :
- un casque (10) doté d'un corps de conteneur (11), dans lequel une tête de patient peut être logée, présentant une extrémité ouverte, un collier élastique (12) qui peut être couplé de façon étanche au cou du patient et adapté pour fermer l'extrémité ouverte dudit corps de conteneur (11), un moyen d'arrivée (17) au moins d'un premier gaz respirable dans un volume confiné par le corps de conteneur (11) et par le collier (12), dans lequel le moyen d'arrivée (17) peut être connecté à un premier circuit d'alimentation (170) du premier gaz respirable; et un moyen d'écoulement (18) à partir duquel les gaz expirés par le patient sortent dudit volume ;
- une canule nasale (20) présentant un tube flexible (21) à une première extrémité sur laquelle un distributeur nasal (22) est fixé pour l'administration d'un second gaz respirable directement dans le nez d'un patient, dans laquelle au moins une partie du tube flexible (21) et le distributeur nasal (22) de la canule nasale (20) sont disposés à l'intérieur du volume confiné par le corps de conteneur (11) et par le collier (12) du casque (10), **caractérisé en ce que**
le casque (10) présente une ouverture traversante (190) adaptée pour mettre l'extérieur du casque (10) en communication avec ledit volume intérieur ; au moins une partie de la canule nasale (20) étant connectée de façon étanche à ladite ouverture traversante (190) du casque (10).

2. Système (100) selon la revendication 1, qui comprend un dispositif de raccordement (40) relié de façon étanche à l'ouverture traversante (190) du casque (10) et au tube flexible (21) de la canule nasale (20) pour raccorder le tube flexible (21) de la canule nasale (20) à un second circuit d'alimentation (30) du second gaz respirable disposé à l'extérieur du volume du casque (30).

3. Système (100) selon la revendication 2, dans lequel le dispositif de raccordement (40) est connecté de façon amovible à la canule nasale (20).

4. Système (100) selon l'une quelconque des revendications 2 à 3 précédentes, dans lequel le dispositif de raccordement (40) est relié de façon amovible à l'ouverture traversante (190).

5. Système selon l'une quelconque des revendications 2 à 4 précédentes, dans lequel le dispositif de raccordement (40) est connecté à une seconde extrémité du tube flexible (21), distale par rapport à la première extrémité pourvue du distributeur nasal (22).

6. Système selon la revendication 5, dans lequel la canule nasale (20) est totalement contenue dans le volume confiné par le corps de conteneur (11) et par le collier (12) du casque (10).

7. Système selon la revendication 2, 5 ou 6, dans lequel le dispositif de raccordement (40) est adapté pour être connecté de façon amovible au second circuit d'alimentation (30) prolongeant la seconde extrémité du tube flexible (21) de la canule nasale (20) vers l'extérieur du volume confiné par le corps de conteneur (11) et par le collier (12) du casque (10).

8. Système (100) selon l'une quelconque des revendications 2 à 7 précédentes, dans lequel le dispositif de raccordement (40) comprend :
- un premier raccord (41) adapté pour être connecté de façon étanche à l'ouverture traversante (190) du casque (10) ;
- un second raccord tubulaire (47) adapté pour être disposé à l'intérieur du volume du fluide confiné par le corps de conteneur (11), le collier (12) du casque (10) et le dispositif de raccordement (40), une fois assemblés, et qui est adapté pour être connecté de façon étanche au tube flexible (21) de la canule nasale (20), de préférence à la seconde extrémité de celle-ci ; et
- un troisième raccord tubulaire (48) adapté pour être disposé à l'extérieur du volume du casque (10) et qui est adapté pour être connecté de façon étanche au second circuit d'alimentation (30).

9. Système (100) selon la revendication 8, dans lequel le troisième raccord (48) est de type mâle égal à un connecteur mâle (23) dont la seconde extrémité du tube flexible (21) de la canule nasale (20) est pourvue, et le second raccord (47) est de type femelle et est adapté pour être couplé avec le connecteur mâle (23) dont la seconde extrémité du tube flexible (21) de la canule nasale (20) est pourvue.

10. Système (100) selon la revendication 8 ou 9, dans lequel :
- le corps de conteneur (11) du casque (10) est pourvu d'une bride rigide (19) qui délimite l'ouverture traversante (190) ; et dans lequel
- le premier raccord est défini par une bague de fixation (41) fixée de façon étanche à la bride rigide (19) du corps de conteneur (11) du casque (10).

11. Système (100) selon la revendication 5, dans lequel le dispositif de raccordement (40) comprend une poche de logement (45) adaptée pour loger la seconde extrémité du tube flexible (21), laquelle poche de logement (45) fait saillie géométriquement à l'extérieur du volume délimité par le corps de conteneur (11) du casque (10) et délimite une partie périphérique du volume intérieur confiné par le corps de conteneur (11) et par le collier (12) dans laquelle la tête du patient peut être logée.

12. Système (100) selon la revendication 1, dans lequel le tube flexible (21) est au moins partiellement perméable à une vapeur d'eau contenue dans le second gaz respirable.

13. Système selon la revendication 1, dans lequel le premier gaz respirable est un mélange sec d'air et d'oxygène et le second gaz respirable est un mélange humide de vapeur d'eau et d'oxygène et/ou d'air.

14. Système selon la revendication 1, dans lequel le second gaz respirable est un fluide chaud à une température comprise entre 30 °C et 40 °C.

15. Système selon la revendication 1, dans lequel l'ouverture traversante (190) est formée dans le corps de conteneur (11).

16. Système selon la revendication 1, dans lequel l'ouverture traversante (190) est formée dans le collier (12).
